(19) Europäisches Patentamt
European Patent Office
Office européen des brevets

(11) Veröffentlichungsnummer: **0 165 902**
**B1**

(12) # EUROPÄISCHE PATENTSCHRIFT

(45) Veröffentlichungstag der Patentschrift: **19.04.89**

(51) Int. Cl.⁴: **C 07 J 71/00,** A 61 K 31/585

(21) Anmeldenummer: **85810277.5**

(22) Anmeldetag: **17.06.85**

(54) **Epoxysteroide.**

(30) Priorität: **21.06.84 CH 3009/84**
**28.09.84 CH 4646/84**

(43) Veröffentlichungstag der Anmeldung: **27.12.85 Patentblatt 85/52**

(45) Bekanntmachung des Hinweises auf die Patenterteilung: **19.04.89 Patentblatt 89/16**

(84) Benannte Vertragsstaaten: **AT BE CH DE FR GB IT LI LU NL SE**

(56) Entgegenhaltungen:
**EP-A-0 122 232**
**FR-A-2 370 755**
**US-A-3 013 012**
**US-A-3 095 412**

(73) Patentinhaber: **CIBA- GEIGY AG, Klybeckstrasse 141, CH- 4002 Basel (CH)**

(72) Erfinder: **Biollaz, Michel, Dr., Im Hirshalm 50, CH- 4125 Riehen (CH)**

**EP 0 165 902 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Gegenstand der Erfindung sind neue Steroidverbindungen mit dem Grundgerüst von 20-Spiroxan, und zwar Lactone vom Typ 7α-Acylthio-9α,11α-epoxy-20-spirox-4-en-3,21-dion der Formel I

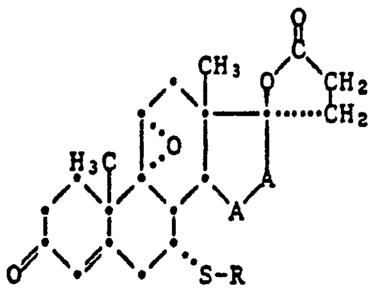

(I)

worin

R ein Alkanoxylrest mit bis zu 4 C-Atomen und -A-A- die Ethylen- oder Cyclopropylen-Gruppe ist. Gegenstand der Erfindung sind weiter auch Verfahren zur Herstellung dieser Verbindungen, diese enthaltende pharmazeutische Zusammensetzungen und deren Herstellung, sowie die therapeutische Anwendung dieser Verbindungen und Zusammensetzungen bei Warmblütern, insbesondere beim Menschen.

Die erfindungsgemässen Verbindungen zeichnen sich durch günstige biologische Eigenschaften aus. Insbesondere weisen sie eine starke Aldosteron-antagonisierende Wirkung auf, indem sie die durch Aldosteron hervorgerufene übermässige Natrium-Retention und Kalium-Exkretion herabsetzen. Deshalb finden sie als Kalium-sparende Diuretika eine wichtige Anwendung in der Therapie von Krankheiten, die mit gestörtem Mineralstoff-Wasser-Gleichgewicht einhergehen, z. B. bei der Behandlung von Herzinsuffizienz, Rhythmusstörungen infolge Kaliummangel, bei Cor pulmonale, Lebercirrhose, Ascites-Prophylaxe, Diabetes mellitus und Hypertonie.

Als Steroide mit Aldosteron-antagonisierender Wirkung werden besonders 20-Spiroxan-Derivate geschätzt, vgl. z. B. Fieser und Fieser: Steroids; Seite 708 (Reinhold Publ. Corp., New York, 1959) und Britische Patentschrift Nr. 1 041 534, darunter in erster Linie das in der Therapie allgemein eingeführte Spironolacton (7α-Acetylthio-20-spirox-4-en-3,21-dion, vgl. Merck Index, 10th Ed., 8610; S. 1254; Merck und Co. Rahway, N.J., U.S.A., 1983. Alle bisher angewendeten Therapeutika dieser Art haben jedoch einen beträchtlichen Nachteil, indem sie immer eine gewisse sexual-spezifische Aktivität besitzen, welche sich bei der üblichen langdauernden Therapie früher oder später störend auswirkt. Besonders unerwünscht sind dabei Störungen, die auf die antiandrogene Wirksamkeit der bekannten Antialdosteron-Präparate zurückzuführen sind.

In der US-Patentschrift 3 095 412 werden 9α,11α-Epoxy-20-spirox-4-en-3,21-dione mit Aldestoron-antagonisierender Wirkung beschrieben, die in 7-Stellung unsubstituiert sind. Über eine antiandrogene Wirksamkeit dieser Verbindungen werden keine Angaben gemacht.

Durch biologische Prüfung im Dosisbereich von ca. 5 - 50 mg/kg wurde nun gefunden, dass durch die Einführung der 9α,11α-Epoxygruppe in das Molekül von Spironolacton Verbindungen der oben charakterisierten Formel I erhältlich sind, welche überraschenderweise die volle Aldosteron-antagonisierende Wirkung der Grundverbindung besitzen, ohne die unerwünschte Nebenwirkung auf den Sexualhormonhaushalt aufzuweisen. So entfaltet z. B. das 7α-Acetylthio-9α,11α-epoxy-20-spirox-4-en-3,21-dion eine analog starke Aldosteron-antagonisierende Wirkung wie Spironolacton (peroral in Kagawa-Test mit adrenalektomierten männlichen Ratten), wogegen eine antiandrogene Wirkung auch in wesentlich erhöhten Dosen in keiner Versuchsanordnung nachgewiesen werden konnte. Das Symbol R der oben charakterisierten Formel I zeitet sich von einer in der Steroidchemie gebräuchlichen insbesondere geradkettigen Alkansäure mit 1 - 4 C-Atomen, vor allem der Essigsäure ab.

Der Cyclopropylenrest als Symbol -A-A- ist vorzugsweise β-orientiert, d. h. so, daßer die 15β, 16β-Methylengruppe bildet.

Bevorzugte Verbindungen der Formel I sind beispielsweise 7α-Acetyl-thio-9α,11α-epoxy-20-spirox-4-en-3,21-dion, und dessen 15β,16β-Methylenanaloges.

Die eingangs charakterisierten Verbindungen der Formel I können durch an sich bekannte Verfahren hergestellt werden, z. B. dadurch, dass man

a) in einer entsprechenden 6,7-ungesättigten 9α,11α-Epoxy-Verbindung der Formel II

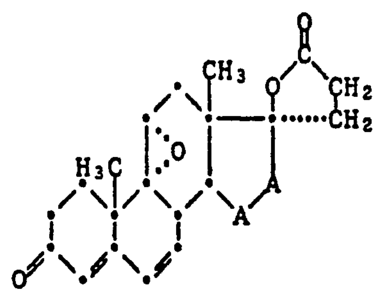

(II) ,

worin -A-A- die oben angegebene Bedeutung hat, an die 6,7-Doppelbindung eine Thiosäure R-SH (III), worin R die oben angegebene Bedeutung hat, anlagert, oder

b) eine entsprechende 9(11)-ungesättigte Verbindung der Formel IV

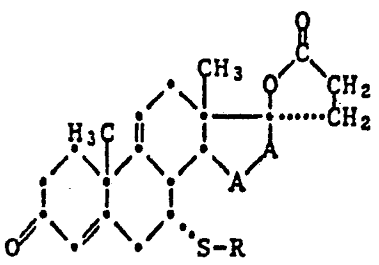

(IV) ,

worin

R und -A-A-, die eingangs angegebene Bedeutung haben, die 9(11)-Doppelbindung epoxidiert.

Die Anlagerung gemäss Verfahrensvariante a) erfolgt in an sich bekannter Weise: so erhitzt man vorzugsweise die betreffende 6,7-Dehydroverbindung (II) mit einem kleinen Überschuss der Thiocarbonsäure (Niederalkanthiosäure) der Formel (III), gegebenenfalls unter Bestrahlung mit ultraviolettem Licht und/oder Säurekatalyse (z. B. in Gegenwart einer organischen Sulfonsäure, wie einer aromatischen Sulfonsäure vom Typ p-Toluol- oder Benzolsulfonsäure) in einem Lösungsmittel, insbesondere einem Niederalkanol, vorzugsweise Methanol. Obwohl die Reaktion bereits bei Raumtemperatur oder wenig oberhalb davon verläuft, sind Temperaturen von etwa 50° bis etwa 80° C bevorzugt; demnach ist es vorteilhaft, im Falle niedriger siedender Lösungsmittel, z. B. insbesondere des Methanols, bei der Siedehitze umzusetzen; die Reaktionstemperatur soll etwa 90 - 100° C nicht übersteigen. Die notwendigen Reaktionszeiten können sich bis zu einigen Stunden erstrecken, sollen jedoch für optimale Resultate am Minimum gehalten werden. Vornehmlich wird unter einem inerten Gas, wie Stickstoff oder Argon, umgesetzt. In einem typischen Verfahren kristallisiert das gebildete Produkt direkt aus dem Reaktionsgemisch nach Abkühlen, gegebenenfalls nach vorangehendem Zufügen von Wasser und/oder Abdampfen überschüssigen Lösungsmittels; gewünschtenfalls kann das Produkt aber auch in üblicher Weise, z. B. durch Chromatographie, isoliert bzw. gereinigt werden. Durch die Anlagerung unter diesen Bedingungen entsteht vorwiegend das gewünschte 7α-Isomere.

Die Durchführbarkeit und insbesondere das günstige Resultat des oben geschilderten Verfahrens beruht auf der überraschenden Feststellung dass der empfindliche Epoxy-Ring durch die Acidität der als Reagens anzuwendenden Thiocarbonsäure nicht angegriffen wird.

Das als Ausgangsstoff verwendete 9α,11α-Epoxy-20-spiroxa-4,6-dien-3,21-dion ist bekannt, vgl. J. Med. Chem. 6, 732 - 735 (1963), die analogen Ausgangsstoffe mit der Methylengruppe in 15,16-Stellung sind durch an sich bekannte Analogieverfahren zugänglich.

Auch die Ausgangsstoffe der Formel III sind bekannt oder in einer bekannten Weise zugänglich.

Die erfindungsgemässen Verbindungen der Formel I können auch gemäss Verfahrensvariante b) erhalten werden, indem man in einer entsprechenden 9(11)-ungesättigten Verbindung, d. h. einem 7α-Acylthio-20-spiroxa-4,9(11)-dien-3,21-dion der oben definierten Formel IV die 9(11)-Doppelbindung epoxidiert.

Die Epoxidierung der 9(11)-Doppelbindung, erfolgt in an sich bekannter Weise durch das Behandeln des Ausgangsmaterials der Formel IV mit einem peroxidischen Oxidationsmittel, wie mit Wasserstoffperoxid, vorzugsweise in Anwesenheit eines Nitrils, z. B. Trichloracetonitril, oder inbesondere mit einer Peroxysäure, vorzugsweise einer organischen Peroxysäure, z. B. einer aliphatischen Peroxysäure, wie insbesondere Perameisensäure oder Peressigsäure, oder vorzugsweise einer aromatischen Peroxysäure. Von den letztgenannten verwendet man vorteilhaft die Perbenzoesäure oder eine substituierte Perbenzoesäure, wie m-Chlorperbenzoesäure, p-Nitroperbenzoesäure oder Monoperoxyphthalsäure (Perphthalsäure). Die Reaktion wird vornehmlich in einem inerten organischen Lösungsmittel ausgeführt, z. B. in einem Alkan, wie Pentan, Hexan oder Heptan, einem halogenierten Niederalkan, wie insbesondere Methylenchlorid, Chloroform oder 1,2-Dichlorethan, oder einem offenkettigen oder cyclischen Ether, wie insbesondere Diethylether, Dioxan oder Tetrahydrofuran, oder einem zweckmässigen Gemisch davon. Die Reaktionstemperatur soll in der Regel nicht eine solche übersteigen, bei welcher die spontane Zersetzung des Reaktionsmittels schneller als die Epoxidierungsreaktion verläuft, vornehmlich arbeitet man bei Zimmertemperatur oder vorzugsweise unterhalb dieser bis zu etwa -20° C, insbesondere zwischen -10 bis +10° C.

Ausgangnstoffe der Formel IV, sofern sie nicht bekannt sind, können durch an sich bekannte Analogieverfahren hergestellt werden, z. B. analog der oben beschriebenen Anlagerung einer Niederalkanthiosäure an 20-Spiroxa-4,6,9(11)-trien-3,21-dion, welches seinerseits bekannt, vgl. J. Med. Chem. 6, 732 - 735 (1963), oder im Falle einer 15,16-Methylenverbindung in an sich bekannter Weise zugänglich ist.

Die pharmazeutischen Präparate der vorliegenden Erfindung enthaltend eine Verbindung der Formel I können insbesondere zur Behandlung von Hyperaldosteronismus verschiedenster Formen verwendet werden. Sie enthalten eine wirksame Menge des Wirkstoffes allein oder im Gemisch mit anorganischen oder organischen, festen oder flüssigen, pharmazeutisch verwendbaren Trägerstoffen und, wenn erwünscht, auch mit anderen pharmakologisch bzw. therapeutisch wertvollen Stoffen, und eignen sich insbesondere zu enteralen, z. B. oralen oder rektalen, oder zur

parenteralen Verabreichung.

Ohne eine nähere spezifische Angabe ist unter dem Begriff "Wirkstoff" im ganzen nachfolgenden Text eine Verbindung der Formel I, wie sie eingangs definiert ist, zu verstehen.

Die vorliegende Erfindung betrifft insbesondere pharmazeutische Zusammensetzungen enthaltend als Wirkstoff mindestens eine erfindungsgemässe Verbindung der Formel I in Form einer sterilen und/oder isotonischen wässrigen Lösung, oder aber im Gemisch mit mindestens einem festen oder halbfesten Trägerstoff.

Die vorliegende Erfindung betrifft auch Arzneimittel, sowie insbesondere Arzneimittel in Form von Dosierungseinheiten, welche mindestens eine der erfindungsgemässen Verbindungen allein oder im Gemisch mit einem oder mehreren Trägerstoffen enthalten, insbesondere solche in fester Form.

Die Erfindung betrifft insbesondere Arzneimittel in Form von Tabletten (einschliesalich Lutschtabletten, Granulen und Pastillen), Dragées, Kapseln, Pillen, Ampullen, Trockenvials oder Suppositorien enthaltend den oben definierten Wirkstoff allein oder im Gemisch mit einem oder mehreren Trägerstoffen.

Als eine besondere Form dieser erfindungsgemässen pharmazeutischen Zusammensetzungen und Arzneimittel kommen auch solche in Betracht, die neben einer erfindungsgemässen Aldosteron-antagonisierenden Verbindung der Formel I (die in diesem Zusammenhang als Komponente A bezeichnet wird), noch eine in bezug auf Elektrolyte unspezifische diuretische Komponente B enthalten.

Als solche in bezug auf Elektrolyt-Ausscheidung unspezifische diuretische Komponente B kommen herkömmliche "klassische" Diuretika oder deren Gemische in Betracht, die sowohl durch renale als auch durch extrarenale Wirkung auf Gewebe die Diurese erhöhen, insbesondere Substanzen mit hemmender Wirkung auf die Rückresorption im Tubulus, wie Saluretika oder Ethacrinsäure und deren Analoge. Eine eingehende Zusammenstellung geeigneter Diuretika dieser Art befindet sich z. B. in der US Patentschrift 4 261 985. Insbesondere geeignet als die Eletrolytunspezifische Komponente B sind Benzothiadiazin-Derivate, wie Thiazide und Hydrothiazide, ferner Benzolsulfonamide, Phenoxyessigsäuren, Benzofuran-2-carbonsäuren und 2,3-Dihydro-benzofuran-2-carbonsäuren. Die Elektrolyt-unspezifische Komponente B kann aus einem einzelnen Wirkstoff oder einer zweckmässigen Kombination mehrerer Wirkstoffe bestehen, wobei die Wirkstoffe auch mehreren der genannten Stoffgruppen angehören können. Ganz besonders kommen als Komponente B die folgenden herkömmlichen Diuretika in Betracht: 1-Oxo-3-(3-sulfamyl-4-chlorphenyl)-3-hydroxyisoindolin, 6-Chlor-7-sulfamyl-3,4-dihydro-1,2,4-benzothiadiazin-1,1-dioxid, 3-Cyclo-pentylmethyl-6-chlor-7-sulfamyl-3,4-dihydro-1,2,4-benzothiadiazin-1,1-dioxid, 4-(2-Methylenbutyryl)-2,3-dichlor-phenoxyessigsäure, 4-Thenoyl-2,3-dichlor-phenoxyessigsäure, (1-Oxo-2-methyl-2-phenyl-6,7-dichlor-5-indanyloxy)-essigsäure, 2-Chlor-4-furfurylamino-5-carboxybenzolsulfonamid, 2-Phenoxy-3-butylamino-5-carboxybenzolsulfonamid und 2-Phenoxy-3-[3-(1-pyrrolyl)-propyl]-5-carboxybenzolsulfonamid.

In derartigen erfindungsgemässen pharmazeutischen Zusammensetzungen und Arzneimitteln beträgt das Verhältnis der Komponente A zur Komponente B, bezogen auf die jeweilige mittlere effektive Dosis, etwa 4 : 1 bis etwa 1 : 4, vorzugsweise etwa 3 : 2 bis etwa 2 : 3. Da die mittlere effektive Dosis jeder spezifischen Komponente ein bekannter, oder durch bekannte pharmakologische Testmethoden einfach feststellbarer Wert ist, ist es dem Fachmann leicht möglich, innerhalb der obengenannten Grenzen ein geeignetes Verhältnis beider Komponenten jedem Patienten gemäss seinem spezifischen Leiden, allgemeinen Gesundheitszustand, individueller Ansprechbarkeit und Alter, sowie auch dessen Geschlecht, zu verordnen.

Beispielsweise enthalten derartige Kombinationspräparate pro Dosiseinheit 15 bis 150 mg, insbesondere 20 bis 100 mg einer Verbindung der Formel I als Komponente A und, als Komponente B, beispielsweise 10 -100 mg, insbesondere 25 - 50 mg 2-Chlor-5-[3-hydroxy-1-oxo-isoindolyl-(3)]-benzolsulfonamid oder 4-(2-Methylenbutyryl)-2,3-dichlorphenoxyessigsäure, 5 - 50 mg, insbesondere 12 - 25 mg 6-Chlor-7-sulfamyl-3,4-dihydro-1,2,4-benzothiadiazin-1,1-dioxid oder 2-Chlor-4-furfurylamino-5-carboxybenzolsulfonamid, 2 - 20 mg, insbesondere 5 - 10 mg 2-Phenoxy-3-[3-(1-pyrrolyl)-propyl]-5-carboxybenzolsulfonamid, 0,1 - 1,0 mg, insbesondere 0,25 - 0,5 mg 3-Cyclopentylmethyl-6-chlor-7-sulfamyl-3,4-dihydro-1,2,4-benzothiadiazin-1,1-dioxid oder 2-Phenoxy-3-butylamino-5-carboxybenzolfonamid, 100 - 400 mg, insbesondere 200 mg 4-Thenoyl-2,3-dichlor-phenoxyessigsäure und 5 - 25 mg, insbesondere 10 mg racemische (1-Oxo-2-methyl-2-phenyl-6,7-dichlor-5-indanyloxy)-essigsäure, oder eine halbe Menge der laevo-Form dieser Säure.

Für die Ödembehandlung in einem mittelschweren Fall werden beispielsweise 1 - 3 Dosiseinheiten täglich genommen, die die Wirkstoffe in Gewichtsmengen enthalten, welche an der höheren Grenze der oben erwähnten besonders bevorzugten Dosierung liegen; ein mittelschwerer Fall der essentiellen Hypertonie wird z. B. mit 1 - 3 Dosiseinheiten behandelt, deren Wirkstoffgehalt an der unteren besonders bevorzugten Mengengrenze liegt.

Der Ausdruck "Arzneimittel" ist angewendet zur Bezeichnung von einzelnen abgetrennten Portionen einheitlicher Zusammensetzung, welche für die medizinische Verabreichung

geeignet sind. Der Ausdruck "Arnzneimittel in Form von Dosierungseinheiten" ist in dieser Beschreibung angewendet zur Bezeichnung von einzelnen abgetrennten Portionen einheitlicher Zusammensetzung, welche für die medizinische Verabreichung geeignet sind und je einzeln eine spezifische Menge des erfindungsgemässen Wirkstoffes enthalten, die etwa 0.05 bis etwa 2, vorzugsweise etwa 0,1 bis etwa 1 Tagesdosis entspricht.

Die Trägerstoffe zur Verwendung in den pharmazeutischen Zusammensetzungen (z. B. Granulaten) zur Herstellung von Tabletten, Dragées, Kapseln und Pillen sind z. B. die folgenden:

a) Verdünnungsmittel, z. B. Stärke, Zucker (wie Lactose, Glukose und Saccharose), Mannit, Sorbit und Kieselsäure,

b) Bindemittel, z. B. Carboxymethylcellulose und andere Cellulosederivate, Alginsäure und ihre Salze (wie Natriumalginat), Gelatine, und Polyvinylpyrrolidon,

c) Feuchtigkeitsregulatoren, z. B. Glycerin,

d) Sprengmittel, z. B. Agar-agar, Calciumcarbonat und Natriumbicarbonat,

e) Retardiermittel zur Verlangsamung der Aufnahme des Wirkstoffes, z. B. Paraffin,

f) Beschleuniger der Resorption, z. B. quaternäre Ammoniumverbindungen,

g) oberflächen-aktive Mittel, z. B. Cetylalkohol und Glycerinmonostearat,

h) Adsorptionsmittel, z. B. Kaolin und Bentonit,

i) Fliessregulier- und Schmiermittel, z. B. Talk, Calciumstearat, Magnesiumstearat und feste Polyethylenglykole.

Diese und ähnliche Träger- bzw. Hilfstoffe können auch mehreren der obgenannten Zwecke dienen.

Die Tabletten, Dragées, Kapseln und Pillen enthaltend die obgenannten erfindungsgemässen pharmazeutischen Zusammensetzungen können mit den üblichen Überzügen und Mantelmaterialien versehen werden, denen gewünschtenfalls Farbstoffe oder Pigmente, z. B. zur Identifizierungs- oder Kennzeichnungszwecken, beigemischt werden. Diese Überzüge können auch eine Zusammensetzung haben, welche eine verlangsamte Abgabe des Wirkstoffes ermöglicht; zu diesem Zwecke sind z. B. Wachse und Cellulosepräparate, wie Acetylcellulosephthalat, oder Hydroxypropylmethylcellulosephthalat, geeignet.

Diese Zusammensetzungen können auch in die Form der Mikrokapseln verarbeitet werden.

Die erfindungsgemässen pharmazeutischen Zusammensetzungen enthalten vorzugsweise von etwa 0,1 bis zu etwa 99,5, insbesondere von etwa 1 bis zu etwa 90 Gewichts-% des Wirkstoffes.

Die empfohlene Tagesdosis des Wirkstoffes der Formel I für einen 75 kg schweren Warmblüter beträgt etwa 30 - 300 mg, vorzugsweise 50 - 150 mg, sie kann jedoch diese Grenzen in Abhängigkeit von Species, Alter und der individuellen Ansprechbarkeit sowohl nach unten wie auch oben weit überschreiten.

Die Herstellung der obgenannten erfindungsgemässen pharmazeutischen Zusammensetzungen, Präparaten, Arzneimitteln und Arzneimiteln in Form von Dosierungaeinheiten erfolgt mittels konventioneller, an sich bekannter Herstellungsverfahren der pharmazeutischen Industrie, z. B. mittels üblicher Misch-, Granulier-, Tablettier-, Dragier-, Lösungs- und Lyophilisierungsverfahren, wobei gewünschtenfalls unter keimfreien Bedingungen gearbeitet wird oder ein Zwischenprodukt oder ein fertiges Produkt sterilisiert wird.

Die vorliegende Erfindung betrifft auch die Verbindungen der Formel I zur Bekämpfung von verschiedensten Formen des Hyperaldosteroismus im Mensch und anderen Warmblütern.

Die erfindungsgemässen Wirkstoffe werden dabei enteral, z. B. rektal oder vor allem oral, oder parenteral, wie insbesondere intravenös, verabreicht. Die erfindungsgemässe Verbindung der Formel I als die Aldosteronantagonisierende Steroid-Komponente A kann mit einer in bezug auf die Elektrolytausscheidung unspezifischen diuretischen Komponente (Komponente B) gleichzeitig bzw. gemeinsam, insbesondere in Form einer entsprechenden pharmazeutischen Zusammensetzung oder eines Arzneimittels, verabreichtht werden.

In den folgenden Beispielen, welche die Erfindung weiter illustrieren, ohne sie dabei einzuschränken, sind die Temperaturen in Celsiusgraden angegeben. Alle Schmelzpunkte sind unkorrigiert.

**Beispiel 1:**

Eine Lösung von 6,5 g 9α,11α-Epoxy-20-spiroxa-4,6-dien-3,21-dion in 275 ml Methanol und 11 ml Thioessigsäure wird während 3,5 Stunden unter Rückfluss unter Argon gekocht, auf etwa 1/3 durch Abdestillieren des Lösungsmittels bei atmosphärischem Druck eingeengt, und abgekühlt. Das Reaktionsprodukt, das aus dem Gemisch kristallisiert, wird abgenutscht, und der durch Einengen der Mutterlauge erhaltener Rückstand über Kieselgel chromatographiert. Die Elution mit einem Gemisch von Hexan-Aceton (3 : 1) ergibt einen weiteren einheitlichen Anteil des gewünschten Produktes. Das erhaltene 7α-Acetylthio-9α,11α-epoxy-20-spirox-4-en-3,21-dion wird aus Methylenchlorid-Methanol kristallisiert; Smp. 224 (Sintern)-242° (Zersetzung).

Eine alternative Isolierung des Endstoffes kann folgendermassen durchgeführt werden: Das nach dem oben geschilderten Verfahren erhaltene durchreagierte Reaktionsgemisch wird noch heiss mit 10 ml Wasser verdünnt und unter Argon beim atmosphärischen Druck eingeengt, bis 190 ml Destillat aufgefangen wird. Das nach Abkühlen auskristallisierte Produkt wird in der

oben beschriebenen Weise verarbeitet.

In analoger Weise kann man die Thioessigsäure durch die Thiopropion- oder Thiobuttersäure ersetzen und die folgenden Verbindungen erhalten:

a) 7α-Propionylthio-9α,11α-epoxy-20-spirox-4-en-3,21-dion (amorph),bzw.

b) 7α-Butyrylthio-9α,11α-epoxy-20-spirox-4-en-3,21-dion (amorph).

**Beispiel 2:**

In analoger Weise, wie im Beispiel 1 beschrieben, wird 9α,11α-Epoxy-15β,16β-methylen-20-spiroxa-4,6-dien-3,21-dion mit Thioessigsäure in Methanol umgesetzt und gemäss Alternative b) weiterverarbeitet, womit 7α-Acetylthio-9α,11α-epoxy-15β,16β-methylen-20-spirox-4-en-3,21-dion, Smp. 268° (Sintern)-292° (Zersetung) erhalten wird.

Der als Ausgangsstoff verwendete 9α,11α-Epoxy-15β,16β-methylen-20-spiroxa-4,6-dien-3,21-dion kann folgendermassen hergestellt werden:

a) Eine Lösung von 20 g 17α,20;20,21-Bismethylendioxypregn-5-en-3β,11β-diol in 150 ml Pyridin und 150 g Acetanhydrid wird 1 Stunde unter Rückfluss erhitzt. Die abgekühlte Reaktionslösung wird auf 3000 g Eisflocken unter Rühren gegossen und bia zum Auftauen weiter gerührt. Der Niederschlag wird abgenutacht und an der Luft getrocknet; das rohe 3β,11β-Diacetoxy-17α,20;20,21-bis-methylen-dioxy-pregn-5-en wird ohne Reinigung weiterverarbeitet.

b) 20,3 g des luftgetrockneten 3,11-Diacetats wird unter Aussenkühlung mit Eiswasser unter Rühren portionsweise in 71 ml einer Lösung eingetragen, die durch Einleiten, bei ca. 0°, von 141 g gasförmigem Fluorwasserstoff in eine Lösung bestehend aus 100 ml Isopropylalkohol, 48 g Harnstoff und 9,6 ml Wasser vorher zubereitet wird.

Das Reaktionsgemisch wird unter der Eiswasserkühlung 1 Stunde gerührt, vorsichtig in eine eiskalte Lösung von 142 g Natriumsulfit in 1015 ml Wasser gegossen und 20 Minuten gerührt. Das Gemisch wird mit Ethylacetat extrahiert und nacheinander mit gesättigter Natriumchloridlösung, eiskalter verdünnter Salzsäure, eiskalter verdünnter Natronlauge und wieder mit verdünnter Kochsalzlösung gewaschen, getrocknet und im Wasserstrahlvakuum eingedampft. Der Rückstand wird an einer 100-fachen Gewichtsmenge Kieselgel chromatographiert. Durch Elution mit einem Gemisch von Methylenchlorid-Aceton (95 : 5) werden einheitliche Fraktionen erhalten, die nach einmaligem Umlösen aus Methylenchlorid-Methanol-Ether 3β,11β-Diacetoxy-17α,21-dihydroxypregn-5-en-20-on vom Schmelzpunkt 231 - 233° liefern.

c) Eine Lösung von 13,8 g der letztgenannten Verbindung in 207 ml Dioxan wird mit 69 g feinpulverigem Mangandioxid versetzt und 3 Stunden am Rückfluss gekocht. Nach Abkühlen auf Zimmertemperatur wird der feste Anteil durch Abnutschen entfernt und mit Chloroform gut nachgewaschen. Das Filtrat wird eingedampft, in Methylenchlorid gelöst und durch eine 10-fache Gewichtsmenge neutrales Aluminiumoxid filtriert. Durch Abdampfen des Lösungsmittels wird kristallines 3β,11β-Diacetoxyandrost-5-en-17-on erhalten, das nach einmaligem Umkristallisieren aus Methylenchlorid-Petrolether bei 177 - 179° schmilzt.

d) Ein Gemisch von 7,5 g 3β,11β-Diacetoxy-androst-5-en-17-on und 150 mg p-Toluolsulfonsäure in 450 ml Benzol und 7,5 ml Ethylenglykol wird 16 Stunden am Wasserabscheider unter Rückfluss gekocht. Nach Abkühlen wird die Lösung mit Ethylacetat verdünnt und sofort mit 225 ml eiskalter gesättigter Natriumchloridlösung gewaschen. Die organische Phase wird nach Trocknen im Wasserstrahlvakuum eingedampft und das ölige 3β,11β-Diacetoxy-17,17-ethylendioxy-androst-5-en für die nächste Stufe ohne Reinigung verwendet.

e) Zu einer gerührten Suspension von 2,35 g Lithiumaluminiumhydrid in 95 ml Tetrahydrofuran wird bei der Innentemperatur von 5 - 10° eine Lösung von 4,7 g 3β,11β-Diacetoxy-17,17-ethylendioxy-androst-5-en in 140 ml Tetrahydrofuran zugetropft, mit 9 ml Tetrahydrofuran nachgespült, und das Gemisch 12 Stunden unter Rückfluss gekocht. Das Reaktionsgemisch wird bei einer Innentemperatur von max. 5° durch vorsichtiges Zutropfen eines Gemisches von 9 ml Tetrahydrofuran und 14 ml Ethylacetat und anschliessend eines Gemisches von 9 ml Tetrahydrofuran und 14 ml Wasser zersetzt und nach Zugabe von 70 g wasserfreiem Natriumsulfat noch 30 Minuten ohne Kühlung gerührt. Feste Anteile werden durch Abnutschen über eine Kieselgurschicht (Nachwaschen mit Tetrahydrofuran) entfernt, und das Filtrat im Wasserstrahlvakuum eingeengt. Der amorphe Rückstand wird an einer 50-fachen Gewichtsmenge Kieselgel chromatographiert. Durch Elution mit einem Gemisch von Methylenchlorid-Aceton (93 : 7) und Abdampfen des Lösungsmittels wird einheitliches 17,17-Ethylendioxy-androst-5-en-3β,11β-diol erhalten, das nach einmaligem Umlösen aus Methylenchlorid-Ether bei 123 - 125° schmilzt.

f) Eine Lösung von 16,8 g 17,17-Ethylendioxy-androst-5-en-3β,11β-diol in 102 ml Tetrahydrofuran wird mit 36,3 g Pyridinhydrobromidperbromid versetzt und 2 1/2 Stunden bei Zimmertemperatur gerührt. Das Gemisch wird mit 26,9 g Natriumjodid versetzt, weitere 30 Minuten gerührt, nacheinander mit einer Lösung von 36,3 g Natriumthiosulfat in 50,4 ml Wasser und mit 100 ml Pyridin versetzt und nochmals 2 Stunden bei Zimmertemperatur

gerührt. Das Reaktionsgemisch wird mit 100 ml Wasser verdünnt und im Wasserstrahlvakuum bei ca. 45° eingeengt. Der Rückstand wird in Ethylacetat aufgenommen und nacheinander mit gesättigter Kochsalzlösung, eiskalter verdünnter Salzsäure, eiskalter verdünnter Natronlauge und nochmals mit gesättigter Kochsalzlösung gewaschen und über Natriumsulfat getrocknet. Durch Abdestillieren der Lösungsmittel im Wasserstrahlvakuum resultiert ein amorpher Rückstand von rohem 16α-Brom-17,17-ethylendioxy-androst-5-en-3β,11β-diol. Das erhaltene Rohprodukt (13 g) wird in 143 ml Dimethylsulfoxid gelöst, im Laufe von 30 Minuten bei 45° unter Rühren mit einem Gemisch von 7 g Kaliumtert-butoxid in 13 ml Dimethylsulfoxid versetzt und 20 Stunden bei 50° (Badtemperatur) gerührt. Das Gemisch wird auf die Raumtemperatur abgekühlt, mit ca. 1300 ml einer gesättigten Ammoniumchloridlösung verdünnt und in Ethylacetat aufgenommen; die organische Phase wird dreimal mit einer gesättigten Kochsalzlösung gewaschen und über Natriumsulfat getrocknet. Durch Abdestillieren der Lösungsmittel im Wasserstrahlvakuum wird amorphes 17,17-Ethylendioxy-androsta-5,15-dien-3β,11β-diol erhalten, dessen Reinheit für die Weiterverarbeitung genügt.

g) Eine Lösung von 800 mg 17,17-Ethylendioxy-androsta-5,15-dien-3β,11β-diol in 40 ml Aceton wird mit 4 ml einer Lösung von 100 mg p-Toluolsulfonsäure in 10 ml Wasser versetzt und 6 Stunden bei Zimmertemperatur gerührt. Nach dem Verdünnen mit 40 ml Wasser wird das Aceton am Wasserstrahlvakuum abdestilliert, der ölige Rückstand in Chloroform aufgenommen und mit eiskalter gesättigter Natriumhydrogencarbonatlösung gewaschen. Nach Abdampfen der organischen Lösungsmittel wird amorphes 3β,11β-Dihydroxy-androsta-5,16-dien-17-on erhalten, das ohne weitere Reinigung für die nächste Stufe verwendet werden kann.

h) Dimethylsulfoxid (64 ml) wird unter Stickstoffatmosphäre mit 1,52 g 55 - 60 %-igem Natriumhydrid (als Mineralöl-Suspension) und 7,57 g Trimethylsulfoxoniumjodid versetzt und zuerst 30 Minuten bei Zimmertemperatur und anschliessend noch weitere 30 Minuten bei einer Aussentemperatur von 34 - 40° gerührt. Dem auf Zimmertemperatur abgekühlten Gemisch wird 8 g 3β,11β-Dihydroxy-androsta-5,15-dien-17-on zugegeben und mit 26 ml Dimethylsulfoxid nachgespült. Das Reaktionsgemisch wird 3 Stunden bei Zimmertemperatur gerührt, auf 1 Liter eiskalte gesättigte Kochsalzlösung gegossen mit wenig Methylalkohol und Wasser nachgespült, mit verdünnter Salzsäure angesäuert und 30 Minuten gerührt. Das abgeschiedene Öl wird in Ethylacetat aufgenommen und die organische Phase nacheinander mit gesättigter Kochsalzlösung, eiskalter verdünnter Natronlauge und wieder mit gesättigter Kochsalzlösung gewaschen. Nach dem Trocknen werden die Lösungsmittel im Wasserstrahlvakuum abgedampft und das resultierende amorphe 3β,11β-Dihydroxy-15β,16β-methylen-androst-5-en-17-on ohne Reinigung der anschliessenden Acetylierung unterworfen.

i) Eine Lösung von 7,9 g 3β,11β-Dihdroxy-15β,16β-methylen-androst-5-en-17-on in 39,5 ml Pyridin und 39,5 ml Essigsäureanhydrid wird 5 Stunden bei Zimmertemperatur stehen gelassen, mit 800 ml Eiswasser verdünnt und nach einstündigem Stehen mit Ethylacetat extrahiert. Die organische Phase wird nacheinander mit gesättigter Kochsalzlösung, eiskalter verdünnter Salzsäure, eiskalter verdünnter Natronlauge und nochmals mit gesättigter Kochsalzlösung gewaschen, getrocknet und im Wasserstrahlvakuum eingeengt. Durch Chromatographie des Rohproduktes an einer 30-fachen Gewichtsmenge Kieselgel und Elution mit einem Gemisch von Methylenchlorid-Aceton-(98 : 2) wird 3β-Acetoxy-11β-hydroxy-15β,16β-methylen-androst-5-en-17-on erhalten, das nach einmaligem Umlösen aus Methylenchlorid-Ether-Petrolether bei 209 - 211° schmilzt.

j) Eine Lösung von 1,75 g 3β-Acetoxy-11β-hydroxy-15β,16β-methylen-androst-5-en-17-on in 10,5 ml Dimethylformamid und 3,5 ml γ-Collidin wird mit 2,6 ml einer Lösung von 5 Gewichts-% Schwefeldioxid in Methansulfonsäurechlorid versetzt und 20 Minuten gerührt, wobei man die Innentemperatur auf etwa 45° steigen lässt. Das Gemisch samt dem ausgefällten Niederschlag wird auf 17,5 ml Eiswasser unter Rühren gegossen und noch weitere 10 Minuten gerührt. Das ausgeschiedene Öl wird in Ethylacetat aufgenommen und nacheinander mit gesättigter Kochsalzlösung, eiskalter verdünnter Salzsäure, eiskalter verdünnter Natronlauge und nochmals mit gesättigter Kochsalzlösung gewaschen. Nach dem Abdampfen der Lösungsmittel resultiert dünnschichtchromatographisch einheitliches 3β-Acetoxy-15β,16β-methylen-androsta-5,9(11)-dien-17-on, das ohne Reinigung weiterverarbeitet wird.

k) Eine Lösung von 5,2 g 3β-Acetoxy-15β,16β-methylen-androsta-5,9(11)-dien-17-on in 127,5 ml Tetrahydrofuran wird unter Eiswasserkühlung mit 1,78 g Lithiumdraht (Stücke von ca. 5 mm Länge) und danach innert 10 Minuten mit einer Lösung von 12,75 ml cyclischem Ethylenacetal des β-Chlorpropionaldehyds [2-(3-Chlorpropyl)-1,3-dioxolan] in 12,75 ml Tetrahydrofuran tropfenweise versetzt und anschliessend eine Stunde unter Eiskühlung und 16 Stunden bei Zimmertemperatur gerührt. Das Reaktionsgemisch wird mit 330 ml Ethylacetat vesetzt und 45 Minuten gerührt, mit weiterem Ethylacetat verdünnt, nacheinander mit gesättigter Kochsalzlösung, eiskalter verdünnter Salzsäure, eiskalter verdünnter Natronlauge und nochmals mit gesättigter Kochsalzlösung gewaschen, getrocknet und im Wasserstrahlvakuum eingeengt. Das ölige Rohprodukt wird in einem Gemisch von Toluol-Ethylacetat (90 : 10) gelöst und durch eine 10-fache Gewichtsmenge Kieselgel filtriert. Das Filtrat liefert nach Abdampfen der Lösungsmittel

4,84 g amorphe Substanz. Diese wird in 363 ml Chloroform gelöst, mit 242 g saurem Aluminiumoxid (Aktivitäts-Stufe 1) versetzt und 2 1/2 Stunden bei Rückfluss Temperatur gerührt, mit weiteren 363 ml Chloroform verdünnt und noch weitere 5 Minuten gerührt und abgekühlt. Das Gemisch wird über Kieselgur genutscht, der Filterkuchen mit Chloroform nachgewaschen, und das Filtrat im Wasserstrahlvakuum eingeengt. Das erhaltene rohe 21-Carbaldehyd (4 g) wird in 20 ml Methylenchlorid und 80 ml Aceton gelöst, bei 5° während 5 Minuten mit 8 ml einer 8-N-Chrom(VI)-schwefelsäure-Lösung versetzt und 45 Minuten unter Eiskühlung gerührt. Das Gemisch wird mit 80 ml eiskaltem Wasser verdünnt, 10 Minuten ohne Kühlung gerührt und mit Methylenchlorid extrahiert. Die organische Phase wird mit eiskalter gesättigter Natriumhydrogencarbonatlösung gewaschen und getrocknet. Durch Abdestillieren der Lösungsmittel im Wasserstrahlvakuum erhält man kristallines Rohprodukt, welches in einer Lösung in Methylenchlorid durch eine 5-fache Gewichtsmenge neutrales Aluminiumoxid filtriert wird. Durch Abdestillieren des Lösungsmittels aus der Hauptfraktion werden Kristalle erhalten, die nach einmaligem Umlösen aus Methylenchlorid-Ether das 3β-Acetoxy-15β,16β-methylen-20-spiroxa-5,9(11)-dien-21-on vom Smp. 241 - 243° liefern.

l) Zu einer Suspension von 1,9 g 3β-Acetoxy-15β,16β-methylen-20-spiroxa-5,9(11)-dien-21-on in 26,6 ml Chloroform und 190 ml Methylalkohol werden 19 ml einer 1-N Natriumhydroxidlösung zugegeben. Das Gemisch wird 1 Stunde bei Zimmertemperatur gerührt, mit 190 ml Wasser verdünnt und mit je einer Portion von Chloroform und eines Gemisches von Chloroform-Methanol (90 : 10) extrahiert. Die vereinigten organischen Phasen werden nach Trocknen im Wasserstrahlvakuum eingeengt und das kristalline Rohprodukt wird einmal aus Methylenchlorid-Ether-Petrolether umkristallisiert. Das erhaltene 3β-Hydroxy-15β,16β-methylen-20-spiroxa-5,9(11)-dien-21-on schmilzt bei 244 - 246°.

m) Aus einer Suspension von 400 mg 3β-Hydroxy-15β,16β-methylen-20-spiroxa-5,9(11)-dien-21-on in 20 ml Toluol und 3 ml Cyclohexanon werden bei Normaldruck 4 ml Lösungsmittel abdestilliert. Es wird auf eine Innentemperatur von ca. 80° abgekühlt, mit 480 mg Aluminiumisopropylat versetzt und zwei Stunden unter Rückfluss gerührt. Die Lösung wird auf Zimmertemperatur abgekühlt, mit einer Lösung von 0,4 ml Essigsäure in 0,8 ml Toluol versetzt und viermal mit je 5 ml Wasser im Wasserstrahlvakuum zur Trockne abgedampft. Der ölige Rückstand wird in Chloroform aufgenommen, nacheinander mit eiskalter verdünnter Salzsäure, Wasser, eiskalter Natronlauge und nochmals Wasser gewaschen, die organische Phase getrocknet und im Wasserstrahlvakuum abgedampft. Das amorphe Rohprodukt wird auf eine 50-fache Gewichtsmenge Kieselgel aufgetragen und mit einem Gemisch von Methylenchlorid-Aceton (98 : 2) chromatographiert. Das erhaltene 15β,16β-Methylen-20-spiroxa-4,9(11)-dien-3,21-dion schmilzt nach einmaligem Umlösen aus Methylenchlorid-Ether-Petrolether bei 172 - 174°.

Variante A:

An) Eine Lösung von 3,27 g 15β,16β-Methylen-20-spiroxa-4,9(11)-dien-3,21-dion in 16,35 ml Dioxan und 6,54 ml Orthoameisensäuretrimethylester wird mit 0,654 ml einer Lösung von 900 mg p-Toluolsulfonsäure in 10 ml Dioxan und 2 ml Ethylalkohol vermischt, 4 Stunden bei Raumtemperatur gerührt, unter Rühren in 430 ml einer eiskalten 0,2-N-Natriumhydroxidlösung gegossen und 15 Minuten intensiv gerührt. Der Niederschlag wird abgenutscht, mit Wasser gewaschen und auf der Nutsche getrocknet. Das erhaltene rohe 3-Ethoxy-15β,16β-methylen-20-spiroxa-3,5,9(11)-trien-21-on wird in 105 ml Aceton gelöst und nacheinander mit einer Lösung von 1,13 g Natriumacetat (Trihydrat) in 8,84 ml Wasser und, unter Kühlung auf -5°, mit 1,55 g N-Bromacetamid und 1,13 ml Essigsäure behandelt. Das Gemisch wird 30 Minuten bei einer Innentemperatur von ca. -3° und nachher noch 15 Minuten ohne Kühlung gerührt, mit einer Lösung von 0,88 g Kaliumjodid in 17,7 ml Wasser und einer Lösung von 5,58 g Natriumthiosulfat in 17,7 ml Wasser nacheinander versetzt, noch weitere 5 Minuten gerührt und mit 88 ml Wasser verdünnt. Das Gemisch wird mit Chloroform extrahiert und die organische Phase mit eiskalter gesättigter Natriumhydrogencarbonatlösung gewaschen. Durch Trocknen und Einengen der organischen Phase wird der amorphe Rückstand erhalten, der in 78 ml Dimethylformamid gelöst, mit 3,89 g Lithiumcarbonat und 3,89 g Lithiumbromid versetzt und 3 Stunden bei 100° gerührt wird. Das abgekühlte Gemisch wird unter Rühren auf 750 ml Eiswasser gegossen, und der Niederschlag abgenutscht und mit Wasser gewaschen. Der Filterkuchen wird in Chloroform gelöst, mit Natriumsulfat getrocknet und im Wasserstrahlvakuum zur Trockne abgedampft. Die Lösung des erhaltenen Rückstands in Methylenchlorid wird durch eine Säule von neutralem Aluminiumoxid (Aktivität II) filtriert und mit weiteren Anteilen desselben Lösungsmittels eluiert. Die Eluate werden eingeengt und das gewünschte 15β,16β-Methylen-20-spiroxa-4,6,9(11)-trien-3,21-dion in amorpher Form durch Zugabe von Ether gefällt. Das Produkt ist gemäss Dünnschichtchromatographie einheitlich und zur Weiterverarbeitung geeignet.

Ao) Eine Lösung von 100 mg 15β,16β-Methylen-20-spiroxa-4,6,9(11)-trien-3,21-dion in 2 ml Methylenchlorid wird mit 75 mg 90 %-iger m-Chlorperbenzoesäure versetzt und 18 Stunden bei ca. 4° und anschliessend noch 3 Stunden bei Zimmertemperatur stehen gelassen. Nach dem Verdünnen mit Methylenchlorid wird das Gemisch nacheinander mit 10 %-iger Kaliumjodidlösung, 10 %-iger

Natriumthiosulfatlösung, eiskalter gesättigter Natriumhydrogencarbonatlösung und Wasser gewaschen, getrocknet und im Wasserstrahlvakuum eingedampft. Das amorphe Rohprodukt wird über eine Kieselgelsäule chromatographiert. Durch Eluieren mit einem Gemisch von Hexan-Ethylacetat (3 : 2) erhält man das gemünschte 9α,11α-Epoxy-15β,16β-methylen-20-spiroxa-4,6-dien-3,21-dion, Smp. 258° (Sintern)-276° (Zersetung).

Variante B

Bn) Eine Lösung von 415 mg 15β,16β-Methylen-20-spiroxa-4,9(11)-dien-3,21-dion in 8 ml Methylenchlorid wird mit 400 mg m-Chlorperbenzoesäure versetzt und 80 Minuten bei Zimmertemperatur gerührt. Das Reaktionsgemisch wird analog Verfahrensstufe Ao aufgearbeitet und das rohe Produkt auf Kieselgel-Säule chromatographiert. Durch Elution mit Hexan-Ethylacetat (1 : 1, V/V) erhält man das chromatographisch einheitliche 9α,11α-Epoxy-15β,16β-methylen-20-spirox-4-en-3,21-dion, welches nach Kristallisstion aus Methylenchlorid-Diisopropylether bei 264 - 266° schmilzt.

Bo) Eine Lösung von 310 mg der letztgenannten Verbindung in 0,2 ml Methylenchlorid und 2,1 ml einer 0,2N-Lösung von trockenem Chlorwasserstoffgas in Dioxan wird mit 230 mg 2,3-Dichlor-5,6-dicyan-benzochinon versetzt und 45 Minuten bei Zimmertemperatur gerührt. Das Reaktionsgemisch wird durch neutrales Aluminiumoxid filtriert und das Adsorbens mit Methylenchlorid nachgewaschen. Nach Abdestillieren der Lösungsmittel resultiert das gewünschte 9α,11α-Epoxy-15β,16β-methylen-20-spiroxa-4,6-dien-3,21-dion, welches mit dem Produkt der Variante A identisch ist und nach Umlösen aus Methylenchlorid-Diisopropylether bei 254° (Sintern)-275° (Zersetzung) schmilzt.

**Beispiel 3:**

Eine Lösung von 414 mg 7α-Acetylthio-20-spiroxa-4,9(11)-dien-3,21-dion und 320 mg m-Chlorperbenzoesäure in 10 ml Dichlormethan wird während 16 Stunden bei 5° stehen gelassen. Nach Verdünnen mit einem Gemisch von Dichlormethan-Ether (1 : 3) wird die Reaktionslösung nacheinander mit Wasser, einer verdünnten wässrigen Kaliumjodid-Lösung, einer verdünnten wässrigen Natriumthiosulfat-Lösung, und nochmals mit Wasser gewaschen, über Natriumsulfat getrocknet und im Vakuum eingeengt. Der Rückstand wird am Kieselgel (60 g) chromatographiert. Nach einem Vorlauf, enthaltend den nicht umkesetzten Ausgangsstoff, folgen mit Hexan-Ethylacetat (2 : 1) Fraktionen, deren übliche Nacharbeitung (s. Beispiel 1) das gewünschte 7α-Acetylthio-9α,11α-epoxy-20-spirox-4-en-3,21-dion, identisch mit dem Produkt des Beispiels 1, ergibt.

**Beispiel 4:**

Tabletten, enthaltend ca. 50 mg Wirkstoff, z. B. 7α-Acetylthio-9α,11α-epoxy-20-spirox-4-en-3,21-dion, werden wie folgt hergestellt:

Zusammensetzung für 1000 Tabletten

| | |
|---|---|
| Wirkstoff, feinst gemahlen | 50,0 g |
| Puderzucker (Saccharose) | 79,0 g |
| Arabisches Gummi | 4,75 g |
| Sorbit | 3,75 g |
| Talk | 2,5 g |
| Magnesiumstearat | 4,9 g |
| Mineralöl | 0,1 g |
| Carboxymethylcellulose (Na-Salz) | 5,0 g |
| | 150,0 g |

**Herstellung**

Der Wirkstoff wird mit dem Puderzucker und dem arabischen Gummi vermischt, gesiebt und mittels einer etwa 35-prozentigen wässerigen Sorbit-Lösung granuliert. Das Granulat wird durch ein Sieb getrieben, getrocknet, nochmals gesiebt und mit den übrigen Hilfsstoffen (Talk, Magnesiumstearat, Mineralöl und Carboxymethylcellulose-Natriumsalz) innig vermischt. Die Mischung wird in üblicher Weise zu Tabletten von 150 mg verpresst.

**Beispiel 5:**

Dragées, enthaltend ca. 50 mg Wirkstoff (z. B. 7α-Acethylthio-9α,11α-epoxy-158,16β-methylen-20-spirox-4-en-3,21-dion vom Beispiel 2) werden folgendermassen hergestellt:

Zusammensetzung eines Dragéekerns

| | |
|---|---|
| Wirkstoff, mikronisiert | 50,0 mg |
| Maisstärke | 90,0 mg |
| Tricalciumphosphat | 100,0 mg |
| Polyvinylpyrrolidon K 25 | 15,0 mg |
| Magnesiumstearat | 2,0 mg |
| Natriumcarboxymethylcellulose | 33,0 mg |
| | 290,0 mg |

**Herstellung von 50 000 Dragéekernen**

Die Mischung von 2,5 kg Wirkstoff, mikronisiert, 4,5 kg Maisstärke und 5 kg Tricalciumphosphat wird mit einer Lösung von 0,75 kg Polyvinylpyrrolidon K 25 in 5 kg destilliertem Wasser im Wirbelschichtverfahren granuliert. Dem bei 45° C getrockneten und durch ein Sieb von 1 mm Maschenweite gedrückten Granulat werden 0,1 kg Magnesiumstearat und

1,65 kg Natriumcarboxymethylstärke zugemischt und zu gewölbten Tabletten à 290 mg verpresst.

**Herstellung von 6,6 kg Zuckerdragées**

6 kg der Dragéekerne werden in einem Dragierkessel von 45 cm Durchmesser mit einem Zuckersirup (2 Teile Zucker und 1 Gewichtsteil destilliertes Wasser), in dem 1,5 % Polyvinylpyrrolidon K25 und 1 % Polyethylenglykol 6000 gelöst sind sowie 20 % Talk suspendiert ist, bis zu einem Gewicht von 360 mg portionenweise überzogen, dazwischen wird mit Warmluft von ca. 60° getrocknet. Anschliessend wird Zuckersirup (2 Teile Zucker und 1 Teil Wasser) bis zum Endgewicht von 400 mg portionenweise aufgetragen. Die Dragées werden schliesslich mit einer Lösung von 2 % Carnaubawachs in Trichlorethylen geglänzt.

**Beispiel 6:**

Weichgelatinekapseln, enthaltend 50 mg Wirkstoff (siehe Beispiel 4 oder 5) werden folgendermassen hergestelt:

Zusammensetzung einer Weichgelatinekapsel

| | |
|---|---|
| Wirkstoff, mikronisiert | 50,0 mg |
| Sojalecithin | 1,5 mg |
| Bienenwachs | 2,5 mg |
| Pflanzenöl | 110,0 mg |
| Pflanzenöl, partiell hydriert | 54,0 mg |
| | 218,0 mg |

**Herstellung von 100 000 Weichgelatinekapseln**

5,0 kg Wirkstoff, mikronisiert, werden in einer durch Schmelzen hergestellten Mischung von 0,15 kg Sojalecithin, 0,25 kg Bienenwachs, 5,4 kg partiell hydriertem Pflanzenöl und 11 kg Pflanzenöl suspendiert und nach dem Stanzverfahren in Gelatinekapseln gebracht. Die Gelatinehülle besteht aus ca. 71 % Gelatine, ca. 28 % Glycerin (85 %) und ca. 1 % Titandioxid, sowie 0,3 % p-Hydroxybenzoesäurepropylester. Die Kapselgrösse beträgt 4 minims (Form oblong).

**Beispiel 7:**

Filmdragées, enthaltend 100 mg Wirkstoff (siehe Beispiel 4 oder 5) werden folgendermassen hergestellt:

Zusammensetzung eines Filmdragéekerns

| | |
|---|---|
| Wirkstoff, mikronisiert | 100,0 mg |
| Polyethylenglykol 6000 | 52,0 mg |
| Kolloidale Kieselsäure | 5,0 mg |
| Stearinsäure | 3,0 mg |
| | 160,0 mg |

**Herstellung von 10 000 Kernen**

1,0 kg Wirkstoff, mikronisiert, wird mit einer Schmelze von 0,52 kg Polyethylenglykol [bereitet unter Zugabe von 0,05 kg kolloidaler Kieselsäure (spezifische Oberfläche 200 $m^2/g$)] vermischt und nach dem Erkalten durch ein Sieb von 1 mm Maschenbreite gedrückt. Dem Granulat werden 0,03 kg pulverförmiger, im voraus gesiebter Stearinsäure zugemischt und zu schwach gewölbten Tabletten à 160 mg verpresst.

**Herstellung von 30 000 Filmdragées**

4,8 kg Kerne werden in einem Dragierkessel von 45 cm Durchmesser mit einer Lösung von Hydroxypropylmethylcellulose (Viskosität 6 cP, 2 %-ige Lösung in Wasser) in destilliertem Wasser, in dem 2 % Talk suspendiert ist, kontinuierlich unter Warmluftzufuhr von 35° besprüht bis auf jedem Kern 5 mg Lack vorhanden ist.

**Beispiel 8:**

Tabletten, enthaltend ca. 50 mg Komponente A und ca. 25 mg Komponente B, werden folgendermassen hergestellt:

Zusammensetzung einer Tablette

| | |
|---|---|
| Komponente A, mikronisiert | 50,0 mg |
| Komponente B, mikronisiert | 25,0 mg |
| Maisstärke | 50,0 mg |
| Kieselsäure, kolloidal | 5,0 mg |
| Gelatine | 5,0 mg |
| Cellulose mikrokristallin | 75,0 mg |
| Natriumcarboxymethylstärke | 20,0 mg |
| Magnesiumstearat | 1,5 mg |
| | 231,5 mg |

**Herstellung von 100 000 Tabletten**

5 kg Komponente A, mikronisiert, 2,5 kg Komponente B, mikronisiert, und 5,0 kg Maisstärke werden mit 0,5 kg kolloidaler Kieselsäure gemischt und mit einer Lösung von 0,5 kg Gelatine in 5,0 kg destilliertem Wasser

(30° C) zu einer feuchten Masse verarbeitet. Diese wird durch ein Sieb von 3 mm Maschenweite getrieben und bei 45° C getrocknet (Wirbelschichttrockner). Das trockene Granulat wird durch ein Sieb von 0,5 mm Maschenweite gedrückt, mit einer im voraus gesiebten Mischung von 7,5 kg mikrokriställiner Cellulose und 2,0 kg Natriumcarboxymethylstärke sowie 0,15 kg Magnesiumstearat gemischt und zu Tabletten von 231,5 mg Gewicht verpresst.

Als Komponente A wird z. B. 7α-Acetylthio-9α,11α-epoxy-20-spirox-4-en-3,21-dion, als Komponente B 6-Chlor-7-sulfamyl-3,4-dihydro-1,2,4-benzothiadiazin-1,1-dioxid verwendet.

In analoger Weise kann man in den entsprechenden Mengen auch die folgenden Wirkstoffe als Komponente B einsetzen:

2-Chlor-5-(3-hydroxy-1-oxo-isoindolyl-3)-benzolsulfonamid oder 4-(2-Methylenbutyryl)-2,3-dichlorphenoxyessigsäure (je 50 mg), 6-Chlor-7-sulfamyl-3,4-dihydro-1,2,4-benzothiadiazin-1,1-dioxid (25 mg), 2-Phenoxy-3-butylamino-5-carboxybenzolsulfonamid (0,5 mg), (1-Oxo-2-methyl-2-phenyl-6,7-dichlor-indanyl-5-oxy)-essigsäure (als Razemat 20 mg, als die laevo-Form 10 mg) oder 3-Cyclopentylmethyl-6-chlor-7-sulfamyl-3,4-dihydro-1,2,4-benzothisdiazin-1,1-dioxid (0,5 mg).

**Beispiel 9:**

Weichgelatinekapseln enthaltend 50 mg Komponente A und 12,5 mg Komponente B werden folgendermassen erhalten:

<u>Zusammensetzung einer Weichgelatinekapsel</u>

| | |
|---|---|
| Komponente A, mikronisiert | 50,0 mg |
| Komponente B, mikronisiert | 12,5 mg |
| Sojalecithin | 1,5 mg |
| Bienenwachs | 2,5 mg |
| Pflanzenöl | 100,0 mg |
| Pflanzenöl, partiell hydriert | 54,0 mg |
| | 220,5 mg |

**Herstellung von 100 000 Weichgelatinekapseln**

6,25 kg eines gleichförmigen Gemisches der Komponenten A und B im Gewichtsverhältnis 4 : 1, mikronisiert, werden in der im Beispiel 5 beschriebenen Weise mit denselben Mengen der Trägermaterislien verarbeitet.

Als Komponente A wird das im Beispiel 8 angegebene Steroid-Wirkstoff, als Komponente B 6-Chlor-7-sulfamyl-3,4-dihydro-1,2,4-benzothiadiazin-1,1-dioxid verwendet.

In analoger Weise kann man in den entsprechenden Mengen auch die folgenden Wirkstoffe als Komponente B einsetzen:

3-Cyclopentylmethyl-6-chlor-7-sulfamyl-3,4-dihydro-1,2,4-benzothiadiazin-1,1-dioxid (0,25 mg), 4-Thienyl-2,3-dichlorphenoxyessigsäure (125 mg), 2-Chlor-5-(3-hydroxy-1-oxo-isoindolyl-3)-benzolsulfonamid (25 mg) oder 2-Chlor-4-furfurylamino-5-carboxybenzolsulfonamid (15 mg).

**Beispiel 10:**

Filmdragées, enthaltend 100 mg Komponente A und 10 mg Komponente B werden folgendermassen hergestellt:

<u>Zusammensetzung eines Filmdragéekerns</u>

| | |
|---|---|
| Komponente A, mikronisiert | 100,0 mg |
| Komponente B, mikronisiert | 10,0 mg |
| Polyethylenglykol 6000 | 52,0 mg |
| Kolloidale Kieselsäure | 5,0 mg |
| Stearinsäure | 3,0 mg |
| | 170,0 mg |

**Herstellung von 10 000 Kernen**

1,1 kg eines gleichmässigen Gemisches der Komponenten A und B im Gewichtsverhältnis 10 : 1, mikronisiert, wird mit einer Schmelze von 0,52 kg Polyethylenglykol [bereitet unter Zugabe von 0,05 kg kolloidaler Kieselsäure (spezifische Oberfläche 200 $m^2/g$)] vermischt und nach dem Erkalten durch ein Sieb von 1 mm Maschenbreite gedrückt. Dem Granulat werden 0,03 kg pulverförmiger, im voraus gesiebter Stearinsäure zugemischt und zu schwach gewölbten Tabletten à 170 mg verpresst.

**Herstellung von 30 000 Filmdragées**

5,1 kg Kerne werden in einem Dragierkessel von 45 cm Durchmesser mit einer Lösung von Hydroxypropylmethylcellulose (Viskosität 6 cP, 2 %-ige Lösung in Wasser) in destilliertem Wasser, in dem 2 % Talk suspendiert ist, kontinuierlich unter Warmluftzufuhr von 35° besprüht, bis auf jedem Kern 5 mg Lack vorhanden ist.

Als Komponente A wird ein Steroid-Wirkstoff gemäss Beispiel 8, als Komponente B 2-Phenoxy-3-[3-(1-pyrrolyl)-propyl]-5-carboxy-benzolsulfonamid verwendet.

In analoger Weise kann man in den entsprechenden Mengen auch die folgende Wirkstoffe als Komponente B einsetzen: 6-Chlor-7-sulfamyl-3,4-dihydro-1,2,4-benzothiadiazin-1,1-dioxid (25 mg), 2-Chlor-5-(3-hydroxy-1-oxo-isoindolyl-3)-benzolsulfonamid (50 mg), 4-(2-Methylen-butyryl)-2,3-dichlorphenoxyessigsäure (50 mg), 2-Chlor-4-furfurylamino-5-carboxybenzolsulfonamid (20 mg), 2-Phenoxy-4-butylamino-5-carboxy-benzolsulfonamid (0,5 mg)

oder 3-Cyclopentylmethyl-6-chlor-7-sulfamyl-3,4-dihydro-1,2,4-benzothiadiazin-1,1-dioxid (0,5 mg).

**Beispiel 11:**

Dragées, enthaltend 40 mg 7α-Acetylthio-9α,11α-epoxy-20-spirox-4-en-3,21-dion als Komponente A und 10 mg 6-Chlor-7-sulfamyl-3,4-dihydro-1,2,4-benzothiadiazin-1,1-dioxid als Komponente B.

Zusammensetzung eines Dragées Kern:

| | |
|---|---|
| Komponente A | 40 mg |
| Milchzucker | 160 mg |
| Stearylalkohol | 77 mg |
| Polyvinylpyrrolidon | 20 mg |
| Magnesiumstearat | 3 mg |
| | 300 mg |

Schutzlack Hülle:

| | |
|---|---|
| 6-Chlor-7-sulfamyl-3,4 dihydro-1,2,4-benzothiadiazin-1,1-dioxid | 10 mg |
| Zucker, Talk, Farbstoff, Bindemittel q.s. ad | 190 mg |
| | 500 mg |

**Herstellung**

Die Steroid-Komponente und Milchzucker werden mit der Stearylalkoholschmelze und einer konzentrierten Polyvinylpyrrolidonlösung granuliert und getrocknet. Die erhaltene Masse wird gesiebt und zu Presslingen von 300 mg Gewicht verpresst. Diese werden mit einer Schutzlackschicht überzogen und sodann mit gefärbtem Zuckersirup, in welchem die diuretische Komponente B gelöst ist, bis zu einem Endgewicht von ca. 500 mg dragiert.

**Beispiel 12:**

Gelatinekapseln enthaltend ca. 50 mg 7α-Acetylthio-9α,11α-epoxy-20-spirox-4-en-3,21-dion als Komponente A und 125 mg 4-Thenoyl-2,3-dichlorphenoxyessigsäure als Komponente B werden folgendermassen hergestellt:

Zusammensetzung einer Trockenkapsel

| | |
|---|---|
| Komponente A | 50,0 mg |
| 4-Thenoyl-2,3-dichlorphenoxy essigsäure | 125,0 mg |
| Milchzucker | 124,0 mg |
| Magnesiumstearat | 1,0 mg |
| | 350,0 mg |

**Herstellung von 10 000 Trockenkapseln**

0,50 kg 7α-Acetylthio-9α,11α-epoxy-20-spirox-4-en-3,21-dion, feinst gemahlen, wird mit 1,25 kg 4-Thenoyl-2,3-dichlorphenoxyessigsäure innig vermischt und nach Bedarf noch zerrieben; zu diesem Gemisch wird 1,24 kg feinst gemahlener Laktose und 0,01 kg Magnesiumstearat durch ein Sieb zugegeben, und homogenisiert. Das Pulver wird gesiebt und in Portionen zu je 350 mg in Gelatinekapseln trocken abgefüllt.

**Patentansprüche** für die Vertragsstaaten: BE, CH, DE, FR, GB, IT, LI, LU, NL, SE

1. Ein 9α,11α-Epoxy-steroid der Formel

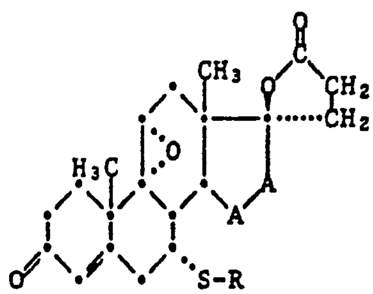

(I) ,

worin R ein Alkanoxylrest mit bis zu 4 C-Atomen und -A-A- die Ethylen- oder Cyclopropylen-Gruppe ist.

2. Eine Verbindung gemäss Anspruch 1, worin R Acetyl ist.

3. Eine Verbindung gemäss Anspruch 1, die 7α-Acetylthio-9α,11α-epoxy-20-spirox-4-en-3,21-dion ist.

4. Eine Verbindung gemäss Anspruch 1, die 7α-Acetylthio-9α,11α-epoxy-15β,16β-methylen-20-spirox-4-en-3,21-dion ist.

5. Eine Verbindung gemäss einem der Ansprüche 1 bis 4 zur Verwendung als Diuretikum.

6. Verfahren zur Herstellung eines im Anspruch 1 definierten 9α,11α-Epoxy-steroids der Formel I, dadurch gekennzeichnet, dass man

a) in einer entsprechenden 6,7-ungesättigten 9α,11α-Epoxy-Verbindung der Formel

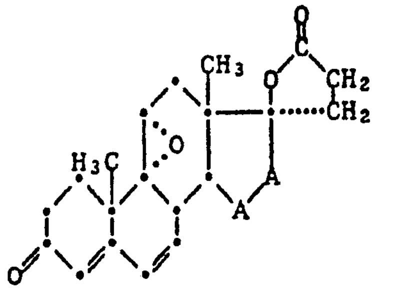

(II) ,

worin -A-A- die im Anspruch 1 angegebene Bedeutung hat, an die 6,7-Doppelbindung eine Thiosäure R-SH(III), worin R die im Anspruch 1 angegebene Bedeutung hat, anlagert, oder

b) eine entsprechende 9(11)-ungesättigte Verbindung der Formel

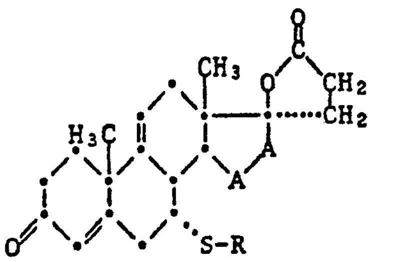

(IV) ,

worin R und -A-A- die im Anspruch 1 angegebene Bedeutung haben, die 9(11)-Doppelbindung epoxidiert.

7. Ein Verfahren gemäss Anspruch 6, dadurch gekennzeichnet, dass man eine der in Ansprüchen 2-4 definierten Verbindungen herstellt.

8. Eine pharmazeutische Zusammensetzung enthaltend als Wirkstoff eine der in Ansprüchen 1 bis 4 definierten Verbindungen und gewünschtenfalls zusätzlich noch ein konventionelles, in bezug auf Elektrolyte unspezifisches Diuretikum (Komponente B).

9. Anwendung einer Verbindung der Formel I gemäss einem der Ansprüche 1 - 4 zur Herstellung einer pharmazeutischen Zusammensetzung gemäss Anspruch 8.

10. Anwendung gemäss Anspruch 9, dadurch gekennzeichnet, dass die Zussmmensetzungen zur Bekämpfung von Hyperaldosteronismus im Menschen und anderen Warmblütern bestimmt sind.

**Patentansprüche** für den Vertragsstaat: AT

1. Verfahren zur Herstellung von einem 9α,11α-Epoxy-steroid der Formel

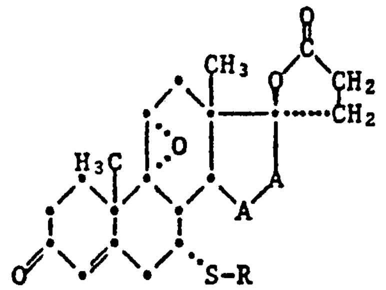

(I)

worin R ein Alkanoylrest mit bis zu 4 C-Atomen und -A-A- die Ethylen- oder Cyclopropylen-Gruppe ist, dadurch gekennzeichnet, dass man

a) in einer entsprechenden 6,7-ungesättigten 9α,11α-Epoxy-Verbindung der Formel

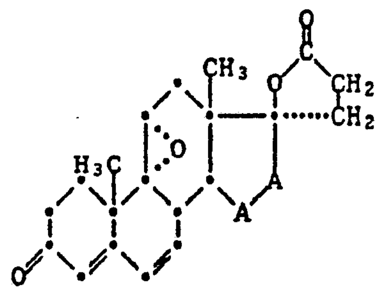

(II) ,

worin -A-A- die oben angegebene Bedeutung hat, an die 6,7-Doppelbindung eine Thiosäure R-SH(III), worin R die oben angegeben Bedeutung hat, anlagert, oder

b) eine entsprechende 9(11)-ungesättigte Verbindung der Formel

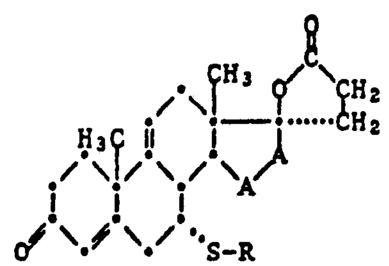

(IV) ,

worin R und -A-A- die oben angegebene Bedeutung haben, die 9(11)-Doppelbindung epoxidiert.

2. Ein Verfahren gemäss Anspruch 1, dadurch

gekennzeichnet, dass man eine Verbindung der Formel I herstellt, worin R Acetyl ist.

3. Ein Verfahren gemäss Anspruch 1, dadurch gekennzeichnet, dass man 7α-Acetylthio-9α,11α-epoxy-20-spirox-4-en-3,21-dion herstellt.

4. Ein Verfahren gemäss Anspruch 1, dadurch gekennzeichnet, dass man 7α-Acetylthio-9α,11α-epoxy-15β,16β-methylen-20-spirox-4-en-3,21-dion herstellt.

5. Ein Verfahren gemäss Anspruch 1, dadurch gekennzeichnet, dass man eine Verbindung gemäss einem der Ansprüche 1 bis 4 zur Verwendung als Diuretikum herstellt.

6. Verfahren zur Herstellung einer pharmazeutischen Zusammensetzung enthaltend als Wirkstoff eine der in Ansprüchen 1 bis 4 definierten Verbindungen und gewünschtenfalls zusätzlich noch eine in bezug auf Elektrolyte unspezifische diuretische Komponente B, dadurch gekennzeichnet, dass man den Wirkstoff (die Wirkstoffe) mit mindestens einem pharmazeutisch anwendbaren Trägermaterial auf nicht-chemischem Wege verarbeitet.

**Claims** for Contracting States: BE, CH, DE, FR, GB, IT, LI, LU, NL, SE

1. A 9α,11α-epoxy steroid of the formula

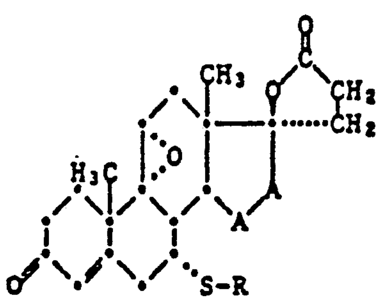

(I)

in which R represents an alkanoyl radical having up to 4 carbon atoms and -A-A- represents an ethylene or cyclopropylene group.

2. A compound according to claim 1 in which R represents acetyl.

3. A compound according to claim 1 that is 7α-acetylthio-9α,11α-epoxy-20-spirox-4-ene-3,21-dione.

4. A compound according to claim 1 that is 7α-acetylthio-9α,11α-epoxy-15β,16β-methylene-20-spirox-4-ene-3,21-dione.

5. A compound according to any one of claims 1 to 4 for use as a diuretic.

6. A process for the manufacture of a 9α,11α-epoxy steroid of the formula I defined in claim 1, characterised in that

a) in a corresponding 6,7-unsaturated 9α,11α-epoxy compound of the formula

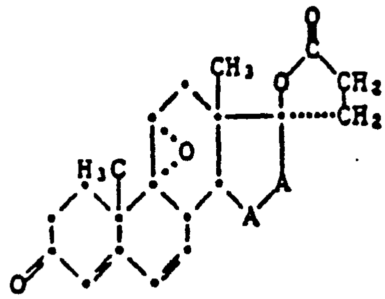

(II),

in which -A-A- has the meaning given in claim 1, a thio acid R-SH (III), in which R has the meaning given in claim 1, is added to the 6,7-double bond, or

b) in a corresponding 9(11)-unsaturated compound of the formula

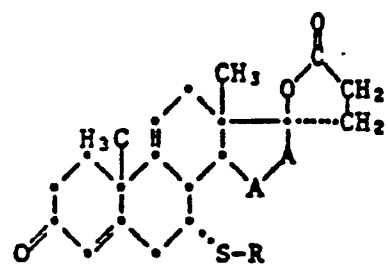

(IV),

in which R and -A-A- have the meanings given in claim 1, the 9(11)-double bond is epoxidised.

7. A process according to claim 6, characterised in that there is manufactured one of the compounds defined in claims 2 to 4.

8. A pharmaceutical composition containing as active ingredient one of the compounds defined in claims 1 to 4 and, if desired, additionally a conventional electrolytenon-specific diuretic (component B).

9. The use of a compound of the formula I according to any one of claims 1 to 4 for the manufacture of a pharmaceutical composition according to claim 8.

10. The use according to claim 9, characterised in that the compositions are intended for combating hyperaldosteronism in human beings and other warm-blooded animals.

**Claims** for the Contracting State: AT

1. A process for the manufacture of a 9α,11α-epoxy steroid of the formula

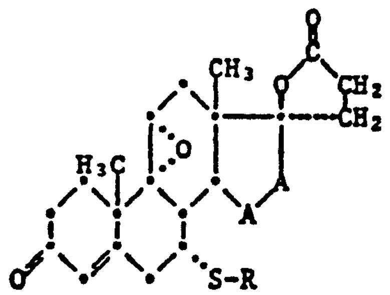

(I)

in which R represents an alkanoyl radical having up to 4 carbon atoms and -A-A- represents an ethylene or cyclopropylene group, characterised in that

a) in a corresponding 6,7-unsaturated 9α,11α-epoxy compound of the formula

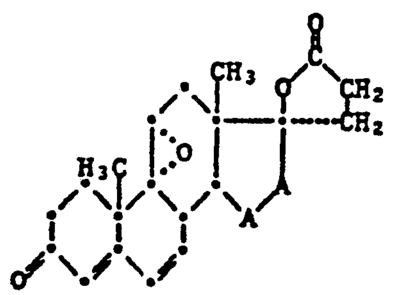

(II),

in which -A-A- has the meaning given above, a thio acid R-SH (III), in which R has the meaning given above, is added to the 6,7-double bond, or

b) in a corresponding 9(11)-unsaturated compound of the formula

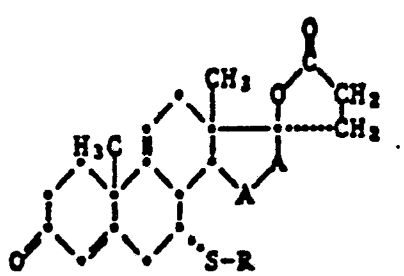

(IV),

in which R and -A-A- have the meanings given above, the 9(11)-double bond is epoxidised.

2. A process according to claim 1, characterised in that a compound of formula I in which R represents acetyl is manufactured.

3. A process according to claim 1, characterised in that 7α-acetylthio-9α,11α-epoxy-20-spirox-4-ene-3,21-dione is manufactured.

4. A process according to claim 1, characterised in that 7α-acetylthio-9α,11α-epoxy-15β,16β-methylene-20-spirox-4-ene-3,21-dione is manufactured.

5. A process according to claim 1, characterised in that a compound according to any one of claims 1 to 4 for use as a diuretic is manufactured.

6. A process for the manufacture of a pharmaceutical composition containing as active ingredient one of the compounds defined in claims 1 to 4 and, if desired, additionally an electrolyte-non-specific diuretic component B, characterised in that the active ingredient (active ingredients) is (are) processed with at least one pharmaceutically acceptable carrier by a non-chemical method.

**Revendications** pour les Etats contractants: BE, CH, DE, FR, GB, IT, LI, LU, NL, SE

1. Un stéroïde 9α,11α-époxy de formule

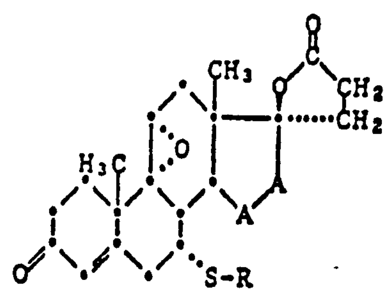

(I),

où R est un reste alcanoyle contenant jusqu'à 4 atomes de C et -A-A- le groupe éthylène ou cyclopropylène.

2. Un composé selon la revendication 1 dans lequel R est l'acétyle.

3. Un composé selon la revendication 1, qui est la 7α-acétylthio-9α,11α-époxy-20-spirox-4-èn-3,21-dione.

4. Un composé selon la revendication 1, qui est la 7α-acétylthio-9α,11α-époxy-15β,16β-méthylène-20-spirox-4-èn-3,21-dione.

5. Un composé selon l'une des revendications 1 à 4 utilisé comme diurétique.

6. Procédé de préparation d'un stéroïde 9α,11α-époxy défini dans la revendication 1, caractérisé en ce que:

a) l'on fixe dans un composé 9α,11α-époxy insaturé en 6,7 correspondant de formule

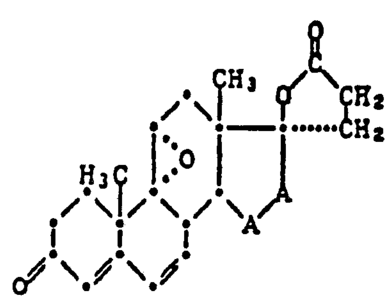

(II),

où -A-A- a la signification donnée dans la revendication 1, sur la double liaison 6,7 un

thioacide R-SH (III), où R a la signification donnée dans la revendication 1, ou

b) l'on époxyde dans un composé insaturé en 9(11) correspondant de formule

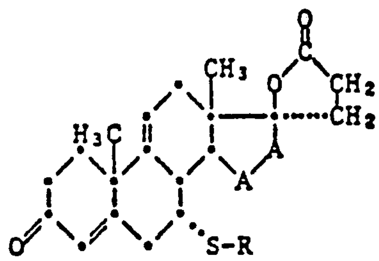

(IV),

où R et -A-A- ont la signification donnée dans la revendication 1, la double liaison 9(11).

7. Un procédé selon la revendication 6, caractérisé en ce que l'on prépare l'un des composés définis dans les revendications 2 - 4.

8. Une composition pharmaceutique contenant comme substance active l'un des composés définis dans les revendications 1 à 4 et éventuellement, en outre, un diurétique classique, non spécifique vis-à-vis de l'électrolyte (composant B).

9. Application d'un composé de formule I selon l'une des revendications 1 - 4 à la préparation d'une composition pharmaceutique selon la revendication 8.

10. Application selon la revendication 9, caractérisée en ce que les compositions sont destinées à la lutte contre l'hyperaldostéronisme chez l'homme et d'autres animaux à sang chaud.

**Revendications** pour l'Etat contractant: AT

1. Procédé de préparation d'un stéroïde 9α,11α-époxy de formule

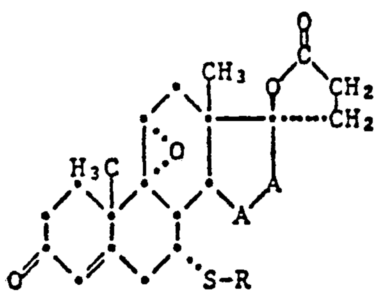

(I),

où R est un reste alcanoyle contenant jusqu'à 4 atomes de C et -A-A- est le groupe éthylène ou le groupe cyclopropylène, caractérisé en ce que:

a) l'on fixe dans un composé 9α,11α-époxy insaturé en 6,7 correspondant de formule

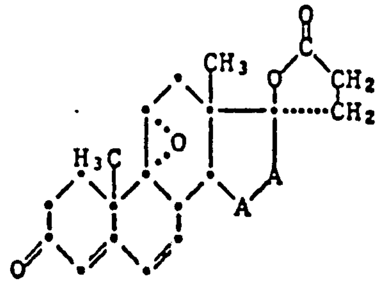

(II),

où -A-A- a la signification indiquée ci-dessus, un thioacide R-SH (III) sur la double liaison 6,7 où R a la signification indiquée ci-dessus, ou

b) l'on époxyde dans un composé insaturé en 9(11) de formule

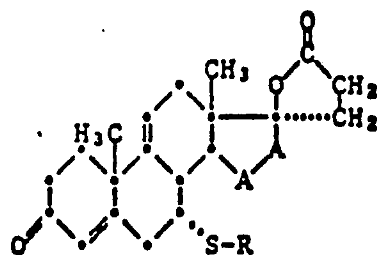

(IV),

où R et -A-A- ont la signification donnée ci-dessus, la double liaison 9(11).

2. Procédé selon la revendication 1, caractérisé en ce que l'on prépare un composé de formule I où R est l'acétyle.

3. Procédé selon la revendication 1, caractérisé en ce que l'on prépare la 7α-acétylthio-9α,11α-époxy-20-spirox-4-èn-3,21-dione.

4. Procédé selon la revendication 1, caractérisé en ce que l'on prépare la 7α-acétylthio-9α,11α-époxy-15β,16β-méthylène-20-spirox-4-èn-3,21-dione.

5. Procédé selon la revendication 1, caractérisé en ce que l'on prépare un composé selon l'une des revendications 1 à 4 utilisé comme diurétique.

6. Procédé de préparation d'une composition, pharmaceutique contenant comme substance active l'un des composés définis dans les revendications 1 à 4 et, éventuellement, en outre, un composant B diurétique non spécifique vis-à-vis de l'électrolyte, caractérisé en ce que l'on traite la substance active (les substances actives) avec au moins un support pharmaceutiquement acceptable par voie non-chimique.